# EUROPÄISCHE PATENTSCHRIFT

(11) **EP 1 037 591 B1**
(45) Veröffentlichungstag und Bekanntmachung des Hinweises auf die Patenterteilung: **12.01.2005**
(21) Anmeldenummer: 98963518.0
(22) Anmeldetag: 26.11.1998
(51) Int. Cl.: A61K 7/06, A61K 7/13, A61K 7/48

(54) **KOSMETISCHE MITTEL ENTHALTEND EINE SPEZIELLE WIRKSTOFFKOMBINATION**
COSMETIC PREPARATIONS CONTAINING A SPECIAL COMBINATION OF ACTIVE AGENTS
AGENTS COSMETIQUES CONTENANT UNE COMBINAISON SPECIALE DE PRINCIPES ACTIFS

(30) Priorität: 05.12.1997 DE 19754053
(43) Veröffentlichungstag der Anmeldung: 27.09.2000
(73) Patentinhaber: Henkel Kommanditgesellschaft auf Aktien, 40589 Düsseldorf (DE)
(72) Erfinder: HÖFFKES, Horst, D-40595 Düsseldorf (DE); MEINIGKE, Bernd, D-51381 Leverkusen (DE); SEIDEL, Kurt, D-40589 Düsseldorf (DE)
(86) Internationale Anmeldenummer: PCT/EP1998/007640
(87) Internationale Veröffentlichungsnummer: WO 1999/029287

(56) Entgegenhaltungen:
- DE-A- 4 403 570
- US-A- 4 898 725
- US-A- 5 585 094

## Beschreibung

Die Erfindung betrifft kosmetische Mittel enthaltend eine Wirkstoffkombination sowie die Verwendung der Wirkstoffkombination in Haut- und insbesondere Haarbehandlungsmitteln, vorzugsweise in Färbe- und/ oder Tönungsmitteln.

Die kosmetische Behandlung von Haut und Haaren ist ein wichtiger Bestandteil der menschlichen Körperpflege. Nicht zuletzt durch die starke Beanspruchung der Haut und Haare durch Umweltbelastungen nimmt die Bedeutung von Pflegeprodukten mit möglichst langanhaltender Wirkung zu.

Sowohl die Reinigung der Haare mit Shampoos als auch die dekorative Gestaltung der Frisur beispielsweise durch Färben oder Dauerwellen sind Eingriffe, die die natürliche Struktur und die Eigenschaften der Haare beeinflussen. So können anschließend an eine solche Behandlung beispielsweise die Naß- und Trockenkämmbarkeit des Haares, der Halt und die Fülle des Haares unbefriedigend sein oder die Haare einen erhöhten Spliß aufweisen.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert.

Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate können noch nicht alle Wünsche der Verbraucher erfüllen.

So ist beispielsweise die biologische Abbaubarkeit der quaternären Ammoniumverbindungen unbefriedigend und ihre mangelnde Kompatibilität mit Aniontensiden schränkt die Formulierungsmöglichkeiten stark ein. Die gleichen Nachteile gelten auch für die kationischen Polymeren. Weiterhin können bei Polymeren unerwünschte Kumulationen auf dem Haar auftreten.

Alkylphosphonate und Alkylphosphate sind zwar mit anionischen Tensiden kompatibel, verfügen aber nur über begrenzte pflegende Eigenschaften.

Auch die kosmetische Pflege der empfindlichen Haut hat in jüngster Zeit an Bedeutung gewonnen, da viele Menschen ihre Haut als empfindlich einstufen, andere durch häufige Einwirkung von Sonnenstrahlung oder von hautreizenden Agenzien ihre Haut belasten, oder an allergischen und entzündlichen Zuständen der Haut erkrankt sind.

Es hat daher nicht an Versuchen gefehlt, durch kosmetische und dermatologische Hautbehandlungsmittel solchen Hautzuständen vorzubeugen oder diese zu heilen. Panthenol sowie Nerzöl sind beispielsweise bekannte Zusätze, die entzündlichen Hautzuständen entgegenwirken.

Es besteht daher weiterhin ein Bedarf nach Wirkstoffen bzw. Wirkstoffkombinationen für kosmetische Mittel mit guten pflegenden Eigenschaften und guter biologischer Abbaubarkeit. Insbesondere in farbstoff- und oder elektrolythaltigen Formulierungen besteht Bedarf an zusätzlichen pflegenden Wirkstoffen, die sich problemlos in bekannte Formulierungen einarbeiten lassen.

DE 4 403 570 offenbart Wirkstoffkombinationen, die Dicarbonsäuren zusammen mit wasser unlöslichen Ölen und Wachsen enthalten.

US 5 585 094 offenbart polysiloxane zur Behandlung der Haare nach der Haarwäsche.

Es wurde nun überraschenderweise gefunden, daß eine Kombination aus bestimmten Dicarbonsäuren, wasserunlöslichen Ölen oder Wachsen und aminosubstituierten Polysiloxanen diese Anforderungen in hervorragender Weise erfüllt.

Gegenstand der Erfindung sind daher kosmetische Mittel enthaltend
(A) eine Dicarbonsäure ausgewählt aus der Gruppe, die gebildet wird durch Verbindungen der allgemeinen Formel (I), 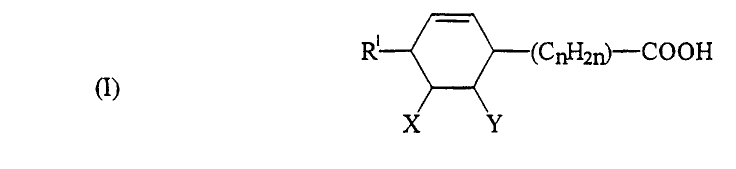 in der R' steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (I), die zusätzlich noch 1 bis 3 Methyl- oder Ethylsubstituenten am Cyclohexenring tragen, Dicarbonsäuren, die aus den Dicarbonsäuren gemäß Formel (I) formal durch Anlagerung eines Moleküls Wasser an die Doppelbindung im Cyclohexenring entstehen, oder einem physiologisch verträglichen Salz einer dieser Säuren,
(B) ein wasserunlösliches Öl oder Wachs,
   und
(C) ein aminosubstituiertes Polysiloxan.

### Dicarbonsäuren der Formel (I) sind in der Literatur bekannt.

Ein Herstellungsverfahren ist beispielsweise der US-Patentschrift 3,753,968 zu entnehmen. Die deutsche Patentschrift 22 50 055 offenbart die Verwendung dieser Dicarbonsäuren in flüssigen Seifenmassen. Aus der deutschen Offenlegungsschrift 28 33 291 sind deodorierende Mittel bekannt, die Zink- oder Magnesiumsalze dieser Dicarbonsäuren enthalten. Schließlich sind aus der deutschen Offenlegungsschrift 35 03 618 Mittel zum Waschen und Spülen der Haare bekannt, bei denen durch Zusatz dieser Dicarbonsäuren eine merklich verbesserte haarkosmetische Wirkung der im Mittel enthaltenen wasserlöslichen ionischen Polymeren enthalten wird. Die dort offenbarten Haarspülmittel können auch wasserunlösliche, kosmetische Öl- und Fettkomponenten enthalten, deren Solubilisierung durch die Dicarbonsäuren erheblich verbessert wird.
Keiner dieser Druckschriften ist aber der geringste Hinweis auf die überraschenden pflegenden Effekte der erfindungsgemäßen Wirkstoffkombination zu entnehmen.

Die Dicarbonsäuren der Formel (I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäß ebenso wie die reinen Verbindungen eingesetzt werden.

Es sind solche Dicarbonsäuren der Formel (I) bevorzugt, bei denen R¹ steht für eine lineare oder methylverzweigte, gesättigte Alkylgruppe mit 4 bis 8 Kohlenstoffatomen und n für eine Zahl von 6 bis 10.

Erfindungsgemäß einsetzbar neben den bevorzugten Dicarbonsäuren gemäß Formel (I) sind auch solche Dicarbonsäuren, die sich von den Verbindungen gemäß Formel (I) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid® 1550 und Westvaco Diacid® 1595 (Hersteller: Westvaco) erhältlich.

Neben den Dicarbonsäuren selbst können auch deren physiologisch verträgliche Salze erfindungsgemäß eingesetzt werden. Beispiele für solche Salze sind die Alkali-, Erdalkali, Zinksalze sowie Ammoniumsalze worunter im Rahmen der vorliegenden Anmeldung auch die Mono-, Di- und Trimethyl-, -ethyl- und -hydroxyethyl-Ammoniumsalze zu verstehen sind. Daneben können jedoch auch mit alkalisch reagierenden Aminosäuren, wie beispielsweise Arginin, Lysin, Ornithin und Histidin, neutralisierte Säuren eingesetzt werden. Die Natrium-, Kalium-, Ammonium- sowie Argininsalze sind bevorzugte Salze. Weiterhin kann es aus Formulierungsgründen bevorzugt sein, die Dicarbonsäurekomponente (A) aus den wasserlöslichen Vertretern, insbesondere den wasserlöslichen Salzen auszuwählen. Das Kaliumsalz der 5- und 6-Carboxy-4-hexyl-2-cyclohexen-1-octansäure, das als wäßrige Lösung unter der Bezeichnung Westvaco Diacid® H-240 kommerziell erhältlich ist, hat sich als besonders geeignet erwiesen.

Die zweite Komponente der erfindungsgemäßen Wirkstoffkombination ist ein **wasserunlösliches Fett oder Öl.** Als wasserunlöslich im Sinne der Anmeldung sind solche Stoffe zu verstehen, deren Löslichkeit in Wasser bei 20 °C 1 g/l oder weniger beträgt. Stoffe mit einer Löslichkeit von 0,1 g/l und weniger sind bevorzugt.

Als wasserunlösliche Fette und Öle kommen beispielsweise natürliche Fette und Öle, Mono-, Di- und Triglyceride von gesättigten und ungesättigten Fettsäuren, Ethylenglykolester von Fettsäuren, Fettsäuren, niedrig ethoxylierte Fettsäuren, Fettalkohole, niedrig ethoxylierte Fettalkohole, Cholesterin und niedrig ethoxylierte Cholesterine, aber auch synthetische Produkte wie beispielsweise Silikonöle in Betracht. Als besonders wirksam haben sich Fettsäuren, niedrig ethoxylierte Fettsäuren, Fettalkohole und niedrig ethoxylierte Fettalkohole erwiesen. Als Komponente (B) können auch entsprechende Mischungen verwendet werden.

Beispiele für Komponenten (B) sind Laurylalkohol, Myristylalkohol, Cetylalkohol, Stearylalkohol, Mischungen von Cetyl/Stearylalkohol, Talgfettalkohol, Kokosfettalkohol, Palmitinsäure, Stearinsäure, Jojobaöl, Paraffinöl und Polydimethylsiloxane.

Langkettige Fettalkohole wie Cetylalkohol und Stearylalkohol, deren Mischungen sowie natürliche Fettalkoholmischungen mit einem hohen Gehalt an diesen Komponenten sind bevorzugte Komponenten (B).

Erfindungsgemäß besonders geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquaternäre Polydimethylsiloxane, Quaternium-80). Mittel, die diese aminosubstituierten Polysiloxane enthalten, weisen sich besonders durch die langanhaltende Wirkung des Pflegeeffektes aus, insbesondere auch in Formulierungen die einen hohen Elektrolytgehalt aufweisen und/ oder Farbstoffe enthalten.

Die Dicarbonsäuren (A) sind in den erfindungsgemäßen Mitteln üblicherweise in Mengen von 0,1 bis 20 Gew.-%, bezogen auf das Mittel, enthalten. In vielen Fällen haben sich Mengen von 1 bis 10 Gew.-% als besonders vorteilhaft erwiesen.

Die wasserunlöslichen Fette und Öle (B) sind in den erfindungsgemäßen Mitteln bevorzugt in Mengen von 0,1 bis 30 Gew.-%, bezogen auf das Mittel, enthalten. Mengen von 1 bis 20, insbesondere jedoch von 3 bis 10 Gew.-% sind besonders bevorzugt.

Die aminosubstituierten Polysiloxane (C) sind in den erfindungsgemäßen Mitteln vorzugsweise in Mengen von 0,1 bis 10, insbesondere 0,5 bis 5 Gew.-% enthalten.

Die erfindungsgemäße Wirkstoffkombination läßt sich problemlos in bekannte Formulierungen einarbeiten und bewirkt eine langanhaltenden Pflegeeffekt.

Neben den obligatorischen Komponenten (A), (B) und (C) können die erfindungsgemäßen kosmetischen Mittel alle für solche Mittel üblichen Komponenten enthalten. Die Auswahl dieser weiteren Komponenten wird im wesentlichen durch die Art des kosmetischen Mittels bestimmt.

Besonders bevorzugt werden zusätzlich wasserlösliche Polymere eingesetzt. Dabei kann es sich im Sinne der vorliegenden Erfindung sowohl um nichtionische, kationische, anionische und/oder amphotere Polymere handeln. Bevorzugte Polymere sind die nichtionogenen Polymere wie beispielsweise Polyvinylpyrrolidon, Vinylpyrrolidon/Vinylacetat-Copolymere und Celluloseether, Stärke und/oder Cyclodextrine. Ebenfalls bevorzugt werden kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol und/oder zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere, eingesetzt.

### Eine weitere Klasse von solchen fakultativen Komponenten stellen die nichtionogenen Tenside dar.

### Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und oligoglycoside und deren ethoxylierte Analoga sowie
- Anlagerungsprodukte von 5 bis 60 Mol Ethylenoxid an Rizinusöl und gehärtetes Rizinusöl.

Bei diesen Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den nichtionogenen Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Alkylpolyglykoside gemäß der Formel RO-(Z)ₓ, in der R steht für einen Alkylrest mit 8 bis 22 Kohlenstoffatomen, Z für einen Mono- oder Oligosaccharid und x für eine Zahl von 1,1 bis 5, sind besonders bevorzugte nichtionogene Tenside. Diese Verbindungen sind durch die folgenden Parameter gekennzeichnet.

Der Alkylrest R enthält 8 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylglykoside mit x-Werten von 1,3 bis 2 sind bevorzugt. Ganz besonders bevorzugt sind Alkylglykoside, bei denen x 1,4 bis 1,6 beträgt.

Die Art der erfindungsgemäßen kosmetischen Mittel unterliegt keinen Beschränkungen. Die erfindungsgemäßen Mittel können sowohl auf Haut und/ oder Haar verbleiben (leaveon), als auch nach einer Einwirkzeit von wenigen Sekunden bis Minuten wieder ausgespült werden (rinse-off). Bevorzugt ist der Einsatz der erfindungsgemäßen Wirkstoffkombination in den sogenannten *rinse-off* Produkten. Beispiele für erfindungsgemäß verwendete Mittel sind Duschlotionen, Duschbäder, Bodylotion, Handcreme, Shampoos, Spülungen, Kuren, Konditioniermittel, Tönungsmittel, Färbemittel, Dauerwellmittel, Fixiermittel, Haarsprays und Fönwellen.

Weitere Komponenten dieser Mittel können dann beispielsweise sein:
- anionische Tenside, wie beispielsweise Fettalkylsulfate und -ethersulfate sowie Alkylethercarbonsäuren,
- kationische Tenside, wie beispielsweise quartäre Ammoniumverbindungen, Amidoamine und quaternisierte Ester und Proteinhydrolysate,
- zwitterionische Tenside, wie beispielsweise Betaine,
- ampholytische Tenside,
- Strukturanten wie Glucose und Maleinsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle und Dimethylisosorbid,
- Lösungsvermittler, wie Ethanol, Isopropanol, Ethylenglykol, Propylenglykol, Glycerin und Diethylenglykol,
- Farbstoffe,
- weitere Substanzen zur Einstellung des pH-Wertes,
- Wirkstoffe wie Panthenol, Allantoin, Pyrrolidoncarbonsäuren und deren Salze, Pflanzenextrakte und Vitamine,
- Lichtschutzmittel,
- Konsistenzgeber wie Zuckerester, Polyolester oder Polyolalkylether,
- Wachse, wie Bienenwachs und Montanwachs,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien,
- Reduktionsmittel wie z.B. Thioglykolsäure und deren Derivate, Thiomilchsäure, Cysteamin, Thioäpfelsäure und α-Mercaptoethansulfonsäure,

Die erfindungsgemäßen Mittel können beispielsweise als wäßrige, alkoholische oder wäßrig-alkoholische Lösungen, Cremes, Gele oder Emulsionen formuliert sein.

Weiterhin betrifft die vorliegende Anmeldung die Verwendung der erfindungsgemäßen Wirkstoffkombination in Hautbehandlungsmitteln. Unabhängig von der Art des Hautbehandlungsmittels haben sich dabei folgende zusätzliche Inhaltsstoffe als besonders vorteilhaft erwiesen:
- Jojobaöl,
- Squalen, Squalan,
- Pflegende Öle, wie beispielsweise Nerzöl,
- Synthetische Ölkomponenten wie beispielsweise Ester langkettiger Carbonsäuren, insbesondere Fettsäuren wie Isopropylmyristat
- Paraffine wie beispielsweise Cetiol® S (Hersteller: Henkel) und
- Ether, insbesondere Dialkylether wie beispielsweise der unter dem Handelsnamen Cetiol® OE erhältliche Dioctylether.

Ein weiterer Gegenstand der vorliegenden Anmeldung betrifft die Verwendung der erfindungsgemäßen Wirkstoffkombination in Haarbehandlungsmitteln

Es hat sich gezeigt, daß praktisch unabhängig von der Art des Haarbehandlungsmittels bestimmte weitere Komponenten die erfindungsgemäße Wirkstoffkombination in ihrer Wirkung besonders vorteilhaft ergänzen.
Als besonders vorteilhaft haben sich Haarbehandlungsmittel erwiesen, die neben der erfindungsgemäßen Wirkstoffkombination **Acyllactylate** der allgemeinen Formel (II) 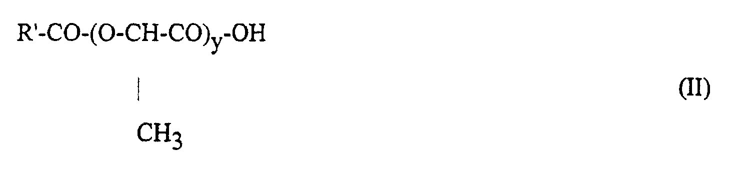 in der R' für eine lineare oder verzweigte, gesättigte oder ungesättigte Alkyl- oder Alkenylgruppe mit 6 bis 22 Kohlenstoffatomen steht und y für eine Zahl vonb 1 bis 5, enthalten.

Acyllactylate, bei denen R für eine, insbesondere gesättigte, lineare oder methylverzweigte Alkylgruppe mit 12 bis 18 Kohlenstoffatomen steht und y für eine Zahl von 1 bis 3, sind besonders bevorzugt.

### Solche Acyllactylate sind im Handel unter dem Warenzeichen Pationic® erhältlich.

Ein Natrium-isostearoyllactyllactylat, das unter der Bezeichnung Pationic® ISL vertrieben wird, ist ein besonders bevorzugtes Acyllactylat.

Weiterhin bevorzugt in erfindungsgemäß verwendeten haarkosmetischen Mitteln einzusetzende Substanzen sind beispielsweise:
- Antischuppenwirkstoffe wie Piroctone Olamine und Zink Omadine,
- haarkonditionierende Verbindungen wie Phospholipide, beispielsweise Sojalecithin, Ei-Lecitin und Kephaline, sowie Silikonöle,
- Oxidationsmittel wie Wasserstoffperoxid, Kaliumbromat und Natriumbromat.

Als besonders vorteilhaft hat sich die erfindungsgemäße Wirkstoffkombination in Mitteln zum Färben und Tönen von Haaren erwiesen. Daher betrifft ein weiterer Gegenstand der vorliegenden Anmeldung die Verwendung der erfindungsgemäßen Wirkstoffkombination in Mitteln zum Färben und Tönen der Haare. Hierbei konnte durch die erfindungsgemäße Wirkstoffkombination trotz des relativ hohen Elektrolyt- und/ oder Farbstoffgehaltes ein langanhaltender Pflegeeffekt erzielt werden.

Solche Mittel enthalten entweder sogenannte "direktziehende" Farbstoffe und/oder Vorprodukte für Oxidationsfarbstoffe.

Übliche **direktziehende Farbstoffe** sind z. B. Nitrobenzolderivate, Andrachinon-Farbstoffe, Triphenylmethanfarbstoffe und Azofarbstoffe. In Haarfärbe- und Tönungsmitteln eingesetzte Vertreter dieser Farbstoffklasse sind beispielsweise die unter den internationalen Bezeichnungen bzw. Handelsnamen HC Yellow 2, HC Yellow 4, Methylgelb, Fluorgelb, Basic Yellow 57, Disperse Orange 3, HC Red 3, HC Red BN, Kardinalrot, Rot Y, Rot X, Basic Red 76, HC Blue 2, Nitroblau, Disperse Blue 3, Basis Blue 99, HC Violet 1, Disperse Violet 1, Disperse Violet 4, Disperse Black 9, Basis Brown 16, Pikraminsäure und Rodol 9 R bekannten Verbindungen. Weitere geeignete direktziehende Farbstoffe sind beispielsweise die Verbindungen 3-Nitro-4-ethylaminobenzoesäure, 1,2,3,4-Tetrahydro-6-nitro-chinoxalin, 2-Nitro-4-amino-4'dimethylaminodiphenylamin-2'-carbonsäure und 2-Nitro-4-aminodiphenylamin-2'carbonsäure.

Als Vorprodukte für Oxidationsfarbstoffe enthalten Haarfärbemittel sogenannte Entwickler- und Kupplerkomponenten. Die Entwicklerkomponenten bilden unter dem Einfluß von Oxidationsmitteln oder Luftsauerstoff untereinander oder unter Kupplung mit einer oder mehreren Kupplerkomponenten die eigentlichen Farbstoffe aus.

Als **Entwicklerkomponenten** werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Spezielle Vertreter sind beispielsweise p-Toluylendiamin, 2,4,5,6-Tetraaminopyrimidin, p-Aminophenol, 2-Aminomethyl-4-aminophenol, 3-Methyl-4-aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5 und 4-Amino-3-methylphenol.

Als **Kupplerkomponenten** werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-methyl-pyrazolon-5, 2,4-Dichlor-3-aminophenol, 1,3-Bis-(2,4-diaminophenoxy)-propan, 2-Chlor-resorcin, 2-Chlor-6-methyl-3-aminophenol und 2-Methylresorcin.

Die folgenden Beispiele sollen den Gegenstand der Erfindung näher erläutern.

### Beispiele

### 1. Untersuchungsverfahren

### 1.1 Gezielte Schädigung von Haarsträhnen

Trockene Haarsträhnen von ca. 2 g Gewicht (Fischbach und Miller Typ 6923) wurden einmal mit 8 g Blondiermischung (Marktprodukt Poly Blond Medium Aufheller) je Strähne eine halbe Stunde lang blondiert. Nach dem Auswaschen der Blondiermischung wurden die Haarsträhnen unmittelbar ohne Zwischentrocknung einer Dauerwellbehandlung mit dem Marktprodukt Poly Lock Normal, je 7g Wellgel bzw. Fixiermittel pro Strähne, unterzogen. Die Einwirkdauern von Wellkomponente und Fixierkomponente betrugen dabei 30 bzw. 15 Minuten. Nach dem Ausspülen der Fixierkomponente wurden die Strähnen getrocknet und mindestens zwei Tage bei Umgebungsbedingungen konditioniert.

### 1.2 Prüfung der Naßkämmbarkeit

Die gespülte Haarsträhne wurde vor der Prüfung mit je 0,2 ml einer 50%igen Lösung von Texapon® N25 (28%ige Lösung von Natriumlaurylethersulfat in Wasser)-Lösung intensiv shampooniert und anschließend ausgespült. Danach wurden 0,5 ml der zu prüfenden Formulierung gleichmäßig in das Haar massiert, eine Minute im Haar belassen und dann sorgfältig ausgespült. Danach wurde mit einem feinzinkigen Kamm gekämmt und der Kämmwiderstand subjektiv beurteilt. Anschließend wurde an derselben Strähne in gleicher Weise eine Standardformulierung als Vergleich geprüft Die Beurteilung wurde entsprechend einem Beurteilungssystem von 1 (= sehr gut) bis 5 (= ungenügend) abgegeben.

### 2. Formulierungen und Ergebnisse

Die Mengenangaben in den folgenden Formulierungen sind jeweils Gew.-%.

### 2.1 Haarspülung

### 2.1.1 gemäß Erfindung

| | |
|---|---|
| Westvaco Diacid® H-240¹ | 10,0 |
| Stenol® 1618² | 5,0 |
| Stärke, löslich | 3,0 |
| Emulsion DC 929³ | 5,0 |
| Parfümöl | 0,15 |
| Euxyl® K400⁴ | 0,1 |
| Wasser ad 100 | |

| | |
|---|---|
| ¹ Kaliumsalz der 4-Hexyl-5(6)-carboxy-2-cyclohexen-1-octansäure (ca. 41 % Aktivsubstanz in Wasser) (WESTVACO) | |
| ² C₁₆/C₁₈-Fettalkohol im Verhältnis 1:1 (HENKEL) | |
| ³ Amodimethicon, Tallowtrimonium Chloride und Nonoxynol-10 35 gew.-%ig in Wasser (DOW CORNING) | |
| ⁴ 1,2-Dibrom-2,4-dicyanobutan und Phenoxyethanol 20:80 (SCHÜLKE & MAYR) | |

### 2.1.2 Vergleich

| | |
|---|---|
| Westvaco Diacid® H-240 | 10,0 |
| Stenol® 1618 | 5,0 |
| Stärke, löslich | 3,0 |
| Parfümöl | 0,15 |
| Euxyl® K400 | 0,1 |
| Wasser ad 100 | |

Die Naßkämmbarkeit der Vergleichsspülung (2.1.2) wurde mit gut, die der erfindungsgemäßen Rezeptur (2.1.1) mit sehr gut (+) bewertet.

Nach mehrmaligem Shampoonieren ergaben sich folgende Bewertungen:

**Tabelle 1**

| Rezeptur | Bewertung der Naßkämmbarkeit nach n Shampoonierungen | | | |
|---|---|---|---|---|
| | n =1 | n =2 | n =3 | n =4 |
| 2.1.1 | 3 | 4 | 4 bis 5 | 5 |
| 2.1.2 | 4 bis 5 | 5 | 5 | 5 |

Es zeigt sich ein besserer Langzeitpflegeeffekt der erfindungsgemäßen Formulierung gegenüber dem Vergleichsbeispiel.

### 2.2 Haartönungsmittel

### 2.2.1 gemäß Erfindung

| | |
|---|---|
| Diacid® H-240 | 10,0 |
| Stenol® 1618 | 5,0 |
| Stärke, löslich | 3,0 |
| 4-Amino-2-nitrodiphenylamin-2'-carbonsäure | 0,1 |
| 6-Nitro-1,2,3,4-tetrahydrochinoxalinhydrochlorid | 0,1 |
| Emulsion DC 929 | 5,0 |
| Wasser | ad 100 |
| mit Ammoniak auf | pH 7,51 |

### 2.2.2 Vergleich

| | |
|---|---|
| Diacid® H-240 | 10,0 |
| Stenol® 1618 | 5,0 |
| Stärke, löslich | 3,0 |
| 4-Amino-2-nitrodiphenyl-amin-2'-carbonsäure | 0,1 |
| 6-Nitro-1,2,3,4-tetrahydro-chinoxalinhydrochlorid | 0,1 |
| Wasser ad 100 | |
| mit Ammoniak auf pH 7,48 | |

Die Bewertung der Naßkämmbarkeit nach unterschiedlicher Anzahl von Shampoonierungen ist in Tabelle 2 dargestellt:

**Tabelle 2**

| Rezeptur | Bewertung der Naßkämmbarkeit nach n Shampoonierungen | | | |
|---|---|---|---|---|
| | n=0 | n=1 | n=2 | n=3 |
| 2.2.1 | 1 | 3 | 4 bis 5 | 5 |
| 2.2.2 | 2 bis 3 | 5 | 5 | 5 |

### 2.3 Haarfärbemittel

### 2.3.1 Erfindungsgemäß

| | |
|---|---|
| Westvaco Diacid® H-240 | 10,0 |
| Plantaren® 600⁵ | 2,0 |
| Hydrenol® D⁶ | 5,0 |
| p-Toluylendiaminsulfat | 0,1 |
| p-Aminophenolhydrochlorid | 0,03 |
| Resorcin | 0,04 |
| m-Aminophenol | 0,004 |
| Emulsion DC 929 | 5,0 |
| Stabilisierungsmittel, Parfümöl, | |
| Puffersalz q.s. | |
| Wasser ad 100 | |

| | |
|---|---|
| Mit Ammoniak wurde ein pH-Wert von 9,5 eingestellt. ⁵ C₁₂₋₁₆-Fettalkahol-1,4-glucosid (CTFA-Bezeichnung: Lauryl Polyglycose; ca. 52 % Aktivsubstanz in Wasser) (HENKEL) | |
| ⁶ C₁₆/C₁₈-Fettalkohol im Verhältnis 1:2 (HENKEL) | |

### 2.3.2 Vergleichsversuch

| | |
|---|---|
| Westvaco Diacid® H-240 | 10,0 |
| Plantaren® 600 | 2,0 |
| Hydrenol® D | 5,0 |
| p-Toluylendiaminsulfat | 0,1 |
| p-Aminophenolhydrochlorid | 0,03 |
| Resorcin | 0,04 |
| m-Aminophenol | 0,004 |
| Stabilisierungsmittel, Parfümöl, | |
| Puffersalz q.s. | |
| Wasser ad 100 | |

Mit Ammoniak wurde ein pH-Wert von 9,5 eingestellt.

Das Haarfärbemittel wurde mit einer kommerziellen 6%igen Wasserstoffperoxiddispersion (Poly Color Brillance Color-Creme, Mischungsverhältnis 1:1) ausgefärbt. Es resulierte eine hellblonde Nuance.

Die Naßkämmbarkeit des mit diesem Färbemittel behandelten Haares wurde nach verschiedener Anzahl von Shampoonierungen wie folgt bewertet:

**Tabelle 3**

| Rezeptur | Bewertung der Naßkämmbarkeit nach n Shampoonierungen | | | |
|---|---|---|---|---|
| | n =0 | n=1 | n =2 | n =3 |
| 2.3.1 | 1 | 3 | 4 bis 5 | 5 |
| 2.3.2 | 2 bis 3 | 5 | 5 | 5 |

### 2.4 Haarshampoo

### 2.3.1 Erfindungsgemäß

| | |
|---|---|
| Westvaco Diacid® H-240 | 5,0 |
| Texapon® N 25⁷ | 5,0 |
| Plantaren® 1200 UP⁸ | 5,0 |
| Akypo® Soft 45 NV⁹ | 10,0 |
| Stenol® 1618 | 3,0 |
| Polymer P1, | |
| gemäß DE 39 29 973¹⁰ | 4,0 |
| Emulsion DC 929 | 5,0 |
| Natrosol® 250 HR¹¹ | 5,0 |
| Parfümöl q.s. | |
| Konservierungsmittel q.s. | |
| Wasser ad 100 | |

| | |
|---|---|
| ⁷ Natriumlaurylethersulfat (ca. 28 % Aktivsubstanz; CTFA-Bezeichnung: Sodium Laureth Sulfate) (HENKEL) | |
| ⁸ C12-C16-Fettalkohol-1,4-glucosid (HENKEL) | |
| ⁹ C₁₂₋₁₄-Fettalkohol-4,5-Ethylenoxid-essigsäure-Natriumsalz (CTFA-Bezeichnung: Sodium Laureth-6 Carboxylate; 21 % Aktivsubstanz in Wasser) (CHEM-Y) | |
| ¹⁰ Polymerisat aus Acrylamidopropyltrimethylammoniumchlorid und Acrylsäure im Molverhältnis 3 : 1 | |
| ¹¹ Hydroxyethylcellulose (HENKEL) | |

Alle Bestandteile wurden bei 60 °C miteinander vermischt. Nach dem Abkühlen erhielt man ein Shampoo von cremiger Konsistenz.

Die Naßkämmbarkeit bei Verwendung des erfindungsgemäßen Shampoos wurde deutlich verbessert und der Pflegeeffekt war auch nach weiterer Shampoonierung mit einem Shampoo aus dem St. d. Technik noch deutlich spürbar.

## Patentansprüche

1. Kosmetische Mittel enthaltend
(A) eine Dicarbonsäure ausgewählt aus der Gruppe, die gebildet wird durch Verbindungen der allgemeinen Formel (I), 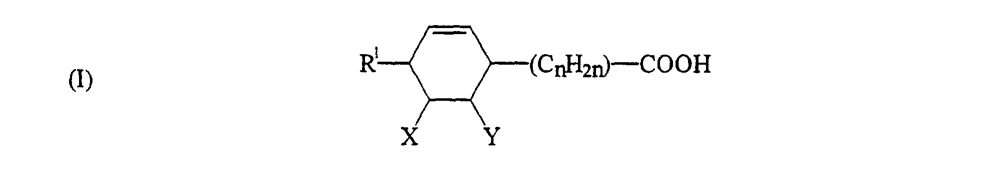 in der R' steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyloder Ethylrest, einer Dicarbonsäure der allgemeinen Formel (I), die zusätzlich noch 1 bis 3 Methyl- oder Ethylsubstituenten am Cyclohexenring tragen, Dicarbonsäuren, die aus den Dicarbonsäuren gemäß Formel (I) formal durch Anlagerung eines Moleküls Wasser an die Doppelbindung im Cyclohexenring entstehen, oder einem physiologisch verträglichen Salz einer dieser Säuren
(B) ein wasserunlösliches Öl oder Wachs,
und
(C) ein aminosubstituiertes Polysiloxan.

2. Mittel nach Anspruch 1, **dadurch gekennzeichnet, daß** R¹ steht für eine lineare oder methylverzweigte, gesättigte Alkylgruppen mit 4 bis 8 Kohlenstoffatomen und n für eine Zahl von 6 bis 10.

3. Mittel nach Anspruch 1, **dadurch gekennzeichnet, daß** die Dicarbonsäure gemäß Formel (I) ein Reaktionsprodukt ist, daß durch Diels-Alder-Reaktion aus Linolsäure und Acrylsäure erhältlich ist.

4. Mittel nach einem der Ansprüche 1 bis 3, **dadurch gekennzeichnet, daß** das physiologisch verträgliche Salz der Dicarbonsäure gemäß Formel (I) ein Alkali- oder Ammoniumsalz ist.

5. Mittel nach einem der Ansprüche 1 bis 4, **dadurch gekennzeichnet, daß** die Komponente (B) ausgewählt ist aus der Gruppe, die von Fettalkoholen, Fettsäuren, ethoxylierten Fettalkoholen und ethoxylierten Fettsäuren gebildet wird.

6. Mittel nach einem der Ansprüche 1 bis 5, **dadurch gekennzeichnet, daß** es sich bei Komponente C um ein Amodimethicon handelt.

7. Mittel nach einem der Ansprüche 1 bis 6, **dadurch gekennzeichnet, daß** die Komponente (A) in Mengen von 0,1 bis 20 Gew.-%, insbesondere von 1 bis 10 Gew.-%, jeweils bezogen auf das gesamte Mittel, enthalten ist.

8. Mittel nach einem der Ansprüche 1 bis 7, **dadurch gekennzeichnet, daß** die Komponente (B) in Mengen von 0,1 bis 15 Gew.-%, insbesondere von 3 bis 10 Gew.-%, jeweils bezogen auf das gesamte Mittel, enthalten ist.

9. Mittel nach einem der Ansprüche 1 bis 8, **dadurch gekennzeichnet, daß** die Komponente (C) in Mengen von 0,1 bis 10, insbesondere 0,5 bis 5 Gew.-%, jeweils bezogen auf das gesamte Mittel, enthalten ist.

10. Mittel nach einem der Ansprüche 1 bis 9, **dadurch gekennzeichnet, daß** das Mittel ein wasserlösliches Polymer, insbesondere Stärke und/oder Cyclodextrine, enthält.

11. Mittel nach einem der Ansprüche 1 bis 10, **dadurch gekennzeichnet, daß** das Mittel ein Acyllactylat enthält.

12. Verwendung eines Mittels nach einem der Ansprüche 1 bis 11 als Haarbehandlungsmittel.

13. Verwendung nach Anspruch 12, **dadurch gekennzeichnet, daß** es sich um ein Färbeund/ oder Tönungsmittel handelt.

14. Mittel nach einem der Ansprüche 1 bis 11 zur Verwendung als Hautbehandlungsmittel.

## Claims

1. A cosmetic preparation containing
(A) a dicarboxylic acid selected from the group consisting of compounds corresponding to general formula (I): 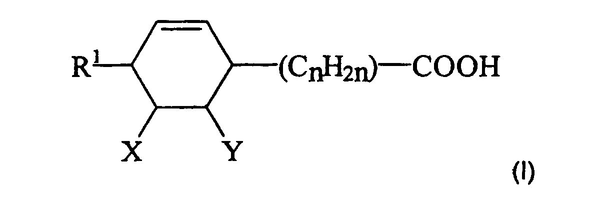 in which R¹ represents a linear or branched alkyl or alkenyl group containing 4 to 12 carbon atoms, n is a number of 4 to 12 and one of the two groups X and Y represents a COOH group and the other represents hydrogen or a methyl or ethyl group,
a dicarboxylic acid corresponding to general formula (I) which additionally contains 1 to 3 methyl or ethyl substituents on the cyclohexene ring, dicarboxylic acids which formally are formed from the dicarboxylic acids (I) by addition of one molecule of water onto the double bond in the cyclohexene ring or a physiologically compatible salt of one of these acids,
(B) a water-insoluble oil or wax and
(C) an aminosubstituted polysiloxane.

2. A preparation as claimed in claim 1, **characterized in that** R¹ is a linear or methyl-branched, saturated alkyl group containing 4 to 8 carbon atoms and n is a number of 6 to 10.

3. A preparation as claimed in claim 1, **characterized in that** the dicarboxylic acid of formula (I) is a reaction product obtainable by DielsAlder reaction from linoleic acid and acrylic acid.

4. A preparation as claimed in any of claims 1 to 3, **characterized in that** the physiologically compatible salt of the dicarboxylic acid of formula (I) is an alkali metal or ammonium salt.

5. A preparation as claimed in any of claims 1 to 4, **characterized in that** component (B) is selected from the group consisting of fatty alcohols, fatty acids, ethoxylated fatty alcohols and ethoxylated fatty acids.

6. A preparation as claimed in any of claims 1 to 5, **characterized in that** component (C) is an amodimethicone.

7. A preparation as claimed in any of claims 1 to 6, **characterized in that** component (A) is present in quantities of 0.1 to 20% by weight and more particularly in quantities of 1 to 10% by weight, based on the preparation as a whole.

8. A preparation as claimed in any of claims 1 to 7, **characterized in that** component (B) is present in quantities of 0.1 to 15% by weight and more particularly in quantities of 3 to 10% by weight, based on the preparation as a whole.

9. A preparation as claimed in any of claims 1 to 8, **characterized in that** component (C) is present in quantities of 0.1 to 10% by weight and more particularly in quantities of 0.5 to 5% by weight, based on the preparation as a whole.

10. A preparation as claimed in any of claims 1 to 9, **characterized in that** it contains a water-soluble polymer, more particularly starch and/or cyclodextrins.

11. A preparation as claimed in any of claims 1 to 10, **characterized in that** it contains an acyl lactylate.

12. The use of the preparation claimed in any of claims 1 to 11 as a hair treatment preparation.

13. The use claimed in claim 12, **characterized in that** the hair treatment preparation is a colouring and/or tinting preparation.

14. The preparation claimed in any of claims 1 to 11 for use as a skin treatment preparation.

## Revendications

1. Produits cosmétiques contenant
(A) un acide dicarboxylique choisi dans le groupe formé par les composés de formule générale (I), 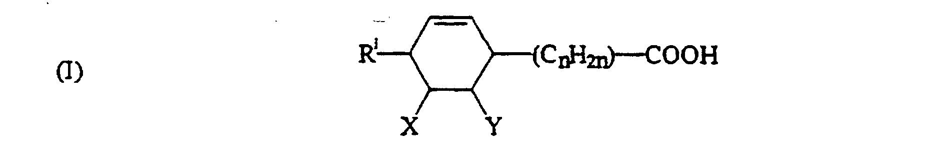 dans laquelle R¹ représente un groupe alkyle ou alcényle linéaire ou ramifié comprenant 4 à 12 atomes de carbone, n représente un nombre de 4 à 12 et un des deux groupes X et Y représente un groupe COOH et l'autre représente un atome d'hydrogène ou un radical méthyle ou éthyle, un acide dicarboxylique de formule générale (I) qui présente en outre encore 1 à 3 substituants méthyle ou éthyle sur le cycle cyclohexène, les acides carboxyliques qui sont formellement formés à partir des acides dicarboxyliques selon la formule (I) par addition d'une molécule d'eau sur la double liaison dans le cycle cyclohexène ou un sel physiologiquement compatible d'un de ces acides
(B) une huile ou une cire insoluble dans l'eau
et
(C) un polysiloxane substitué par amino.

2. Produits selon la revendication 1, **caractérisé en ce que** R¹ représente un groupe alkyle linéaire ou ramifié par un groupe méthyle, saturé, comprenant 4 à 8 atomes de carbone et n représente un nombre de 6 à 10.

3. Produits selon la revendication 1, **caractérisé en ce que** l'acide dicarboxylique selon la revendication (1) est un produit de réaction pouvant être obtenu par une réaction de Diels-Alder à partir d'acide linoléique et d'acide acrylique.

4. Produits selon l'une quelconque des revendications 1 à 3, **caractérisé en ce que** le sel physiologiquement compatible de l'acide dicarboxylique selon la formule (1) est un sel alcalin ou d'ammonium.

5. Produits selon l'une quelconque des revendications 1 à 4, **caractérisé en ce que** le composant (B) est choisi dans le groupe formé par les alcools gras, les acides gras, les alcools gras éthoxylés et les acides gras éthoxylés.

6. Produits selon l'une quelconque des revendications 1 à 5, **caractérisé en ce qu'**il s'agit d'un amodiméthicone pour le composé (C).

7. Produits selon l'une quelconque des revendications 1 à 6, **caractérisé en ce que** le composant (A) est contenu en des quantités de 0,1 à 20% en poids, en particulier de 1 à 10% en poids, à chaque fois par rapport à la totalité du produit.

8. Produits selon l'une quelconque des revendications 1 à 7, **caractérisé en ce que** le composant (B) est contenu en des quantités de 0,1 à 15% en poids, en particulier de 3 à 10% en poids, à chaque fois par rapport à la totalité du produit.

9. Produits selon l'une quelconque des revendications 1 à 8, **caractérisé en ce que** le composant (C) est contenu en des quantités de 0,1 à 10, en particulier de 0,5 à 5% en poids, à chaque fois par rapport à la totalité du produit.

10. Produits selon l'une quelconque des revendications 1 à 9, **caractérisé en ce que** le produit contient un polymère soluble dans l'eau, en particulier des amidons et/ou des cyclodextrines.

11. Produits selon l'une quelconque des revendications 1 à 10, **caractérisé en ce que** le produit contient un lactylate d'acyle.

12. Utilisation d'un produit selon l'une quelconque des revendications 1 à 11 comme produit de traitement pour cheveux.

13. Utilisation selon la revendication 12, **caractérisée en ce qu'**il s'agit d'un produit de teinture et/ou de coloration.

14. Produit selon l'une quelconque des revendications 1 à 11 destiné à une utilisation comme produit de traitement pour la peau.
